# EUROPEAN PATENT APPLICATION

(11) **EP 4 092 101 A1**
(43) Date of publication of application: **23.11.2022**
(21) Application number: 21740845.9
(22) Date of filing: 13.01.2021
(51) Int. Cl.: C12M 3/00, C12N 5/02, C12N 5/074, C12N 5/10

(54) **CELL CULTURE METHOD**

(30) Priority: 14.01.2020 JP 2020003961; 02.06.2020 JP 2020096485
(71) Applicant: AJINOMOTO CO., INC., Chuo-ku Tokyo 104-8315 (JP)
(72) Inventor: OGAWA, Shimpei, Kawasaki-shi, Kanagawa 210-8681 (JP); HIGUCHI, Takuya, Kawasaki-shi, Kanagawa 210-8681 (JP); FUROMITSU, Shumpei, Kawasaki-shi, Kanagawa 210-8681 (JP); NISHIYAMA, Megumi, Kawasaki-shi, Kanagawa 210-8681 (JP); KOSEKI, Kotoe, Kawasaki-shi, Kanagawa 210-8681 (JP)
(74) Representative: Hoffmann Eitle
(86) International application number: PCT/JP2021/000745
(87) International publication number: WO 2021/145320

(57) **Abstract**

A method for culturing a cell, including the following steps:
a first step of preparing a population of cell aggregates having a major axis of not more than 400 µm, and
a second step of suspension culturing the population of cell aggregates obtained in the first step is provided by the present invention.

## Description

### [Technical Field]

The present invention relates to a method for culturing cells. More particularly, the present invention relates to a method for culturing cells, including a step of controlling the size of cell aggregates.

### [Background Art]

Regenerative medicine technology using cells has been receiving high attention in recent years as one of the means capable of treating various diseases and damages that were difficult to treat before. Since regenerative medicine requires a large amount of cells, the development of a method for efficiently culturing cells has been actively conducted. For example, Patent Literature 1 reports a method for culturing stem cells, including treating stem cells with a ROCK inhibitor in a medium.

Stable culture and proliferation of cells essentially requires operations such as regular medium exchange, passaging, and the like. Since such operation involves repetition of complicated processes, there arise problems that the burden on the operator is heavy and that the cell proliferation efficiency and the like are affected by the skill of the operator. For this reason, cell culture devices that monitor the culture environment, such as pH and gas concentration of the medium, and automatically optimize them as necessary (sometimes referred to as "bioreactor" or the like in the present specification), have been developed and utilized in recent years. One example of such bioreactor is, for example, "ambr (registered trade mark)" cell culture device from Sartorius.

### [Citation List]

### [Patent Literature]

### [PTL 1]

JP-A- 2008-099662

### [Summary of Invention]

### [Technical Problem]

The use of bioreactors greatly improves the burden on operators and problems caused by operators during cell culture. On the other hand, however, it is difficult to say at this point that sufficient studies have been made to determine what conditions should be employed to optimize the efficiency of cell growth when using a bioreactor.

Therefore, the purpose of the present invention is to provide, in cell culture using a bioreactor, a method for increasing the efficiency of cell proliferation by a simple and inexpensive means.

### [Solution to Problem]

The present inventors have conducted intensive studies of the above-mentioned problems and found that highly preferable high density culture can be realized by preparing a great number of comparatively small cell aggregates by controlling the size of the aggregates of the cells to be cultured. Based on such finding, they have conducted further studies and completed the present invention.

Accordingly, the present invention provides the following.
[1] A method for culturing a cell, comprising the following steps:
   a first step of preparing a population of cell aggregates having a major axis of not more than 400 µm, and
   a second step of suspension culturing the population of cell aggregates obtained in the first step.
[2] The method of [1], wherein a mean major axis of the cell aggregate in the first step is not more than 110 µm.
[3] The method of [1] or [2], wherein a median major axis of the cell aggregate in the first step is not more than 90 µm.
[4] The method of any of [1] to [3], wherein the first step and the second step are successively performed.
[5] The method of any of [1] to [4], wherein the population of cell aggregates in the first step is prepared using a spinner flask.
[6] The method of any of [1] to [5], wherein a medium used in the second step comprises a calcium ion at a concentration of 0.07 to 0.25 mM.
[7] The method of any of [1] to [3], wherein the population of cell aggregates in the first step is prepared by adjusting a calcium ion concentration and the aforementioned calcium ion concentration is 0.07 to 0.25 mM.
[8] The method of [6] or [7], further comprising setting a magnesium ion concentration to 0.5 to 0.65 mM.
[9] The method of any of [1] to [8], wherein the suspension culture in the second step is performed using a bioreactor.
[10] The method of any of [1] to [9], wherein the cell is a stem cell.
[11] The method of [10], wherein the stem cell is an iPS cell.
[12] A method for culturing cells, comprising a step of suspension culturing a cell in a medium having a calcium ion concentration of 0.07 to 0.25 mM.
[13] The method of [12], wherein the cell is a stem cell.
[14] The method of [13], wherein the stem cell is an iPS cell.

### [Advantageous Effects of Invention]

According to the present invention, preferable high density culture can be realized by highly inexpensive and simple means.

### [Brief Description of Drawings]

[Fig. 1]

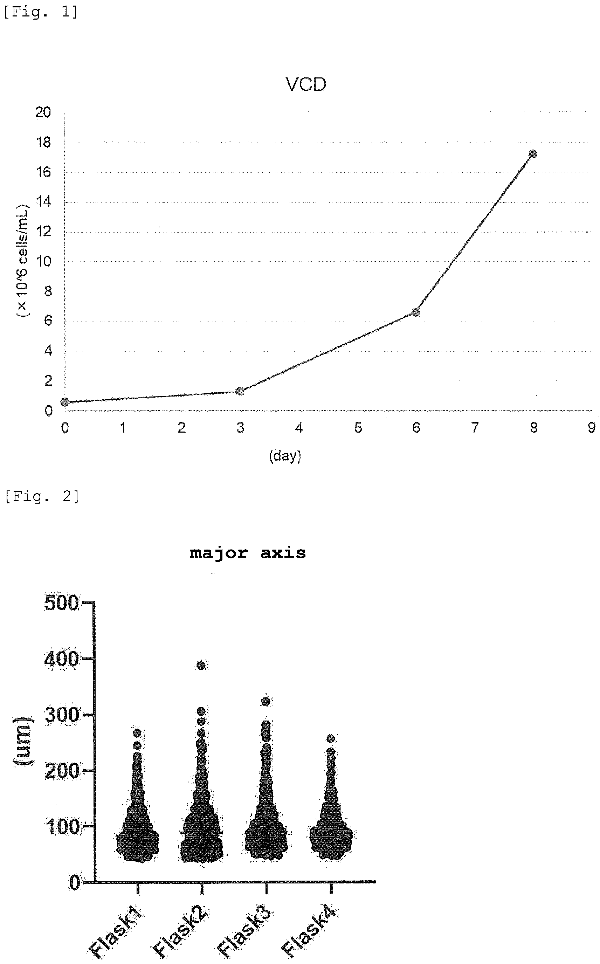

   Fig. 1 shows time-course changes in the number of iPS cells in the present Example.
[Fig. 2]
   Fig. 2 shows size distribution of a population of cell aggregates (at time point of day3 and before suspension culture in a bioreactor, n=4) prepared using a spinner flask in the present Example.
[Fig. 3]

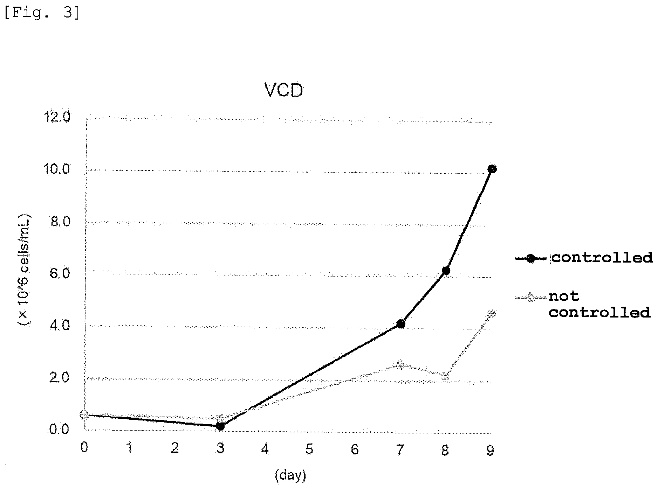

   Fig. 3 shows time-course changes in the number of cells with/without controlling the size of the cell aggregates.
[Fig. 4]

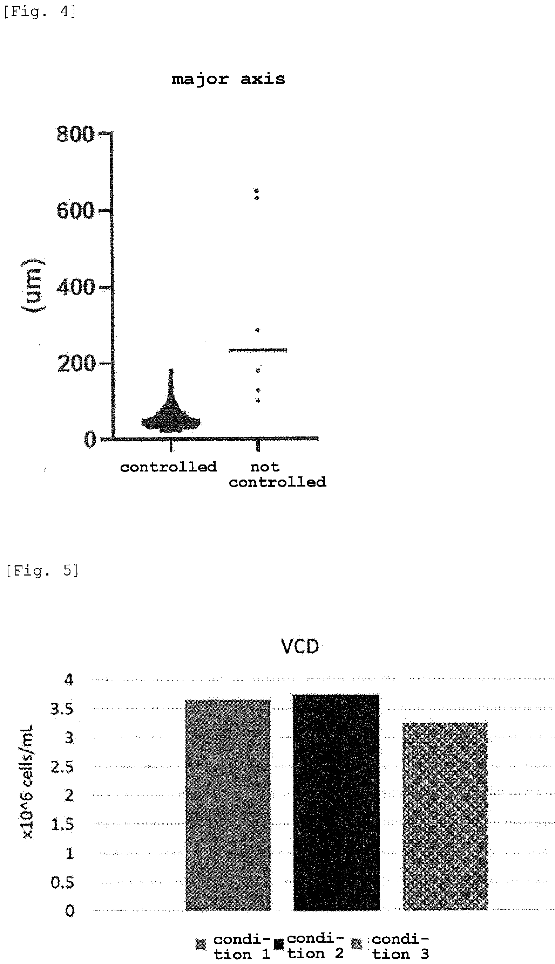

   Fig. 4 shows size distribution of cell aggregates (at time point of day3) with/without controlling the size of the cell aggregates.
[Fig. 5]
   Fig. 5 shows changes in the number of cells when iPS cells were cultured under conditions 1 to 3.
[Fig. 6]

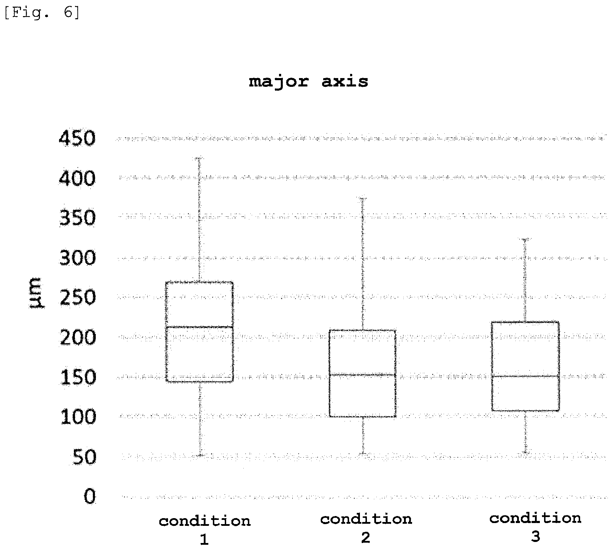

   Fig. 6 shows size distribution of cell aggregates when iPS cells were cultured under conditions 1 to 3.
[Fig. 7]

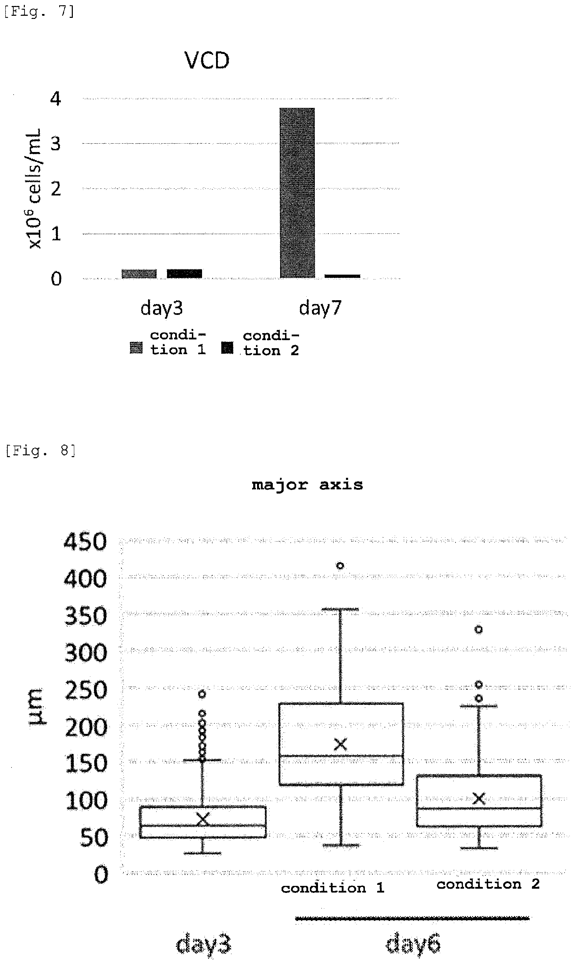

   Fig. 7 shows the influence of the concentrations of Ca²⁺ and Mg²⁺ in the medium on the proliferation of iPS cells.
[Fig. 8]
   Fig. 8 shows the influence of the concentration of Ca²⁺ in the medium on the size of iPS cell aggregates.
[Fig. 9]

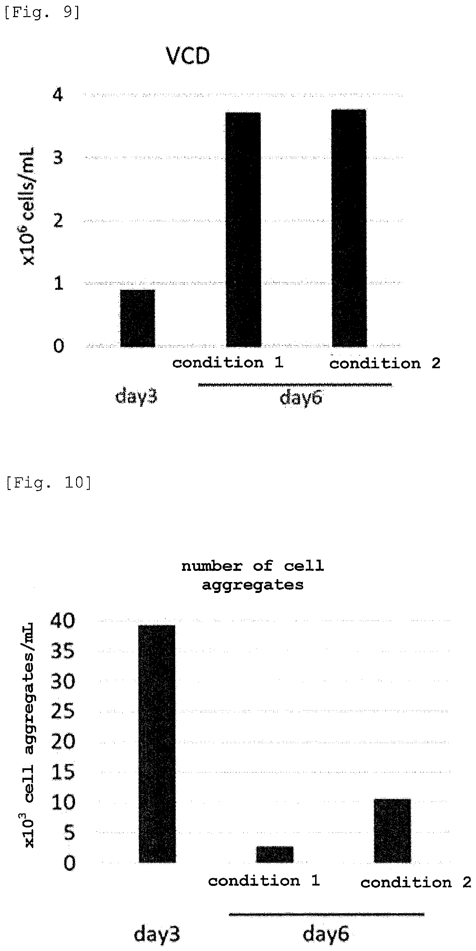

   Fig. 9 shows the influence of the concentration of Ca²⁺ in the medium on the viable cell density (VCD) of iPS cells.
[Fig. 10]
   Fig. 10 shows the influence of the concentration of Ca²⁺ in the medium on the number of iPS cell aggregates.
[Fig. 11]

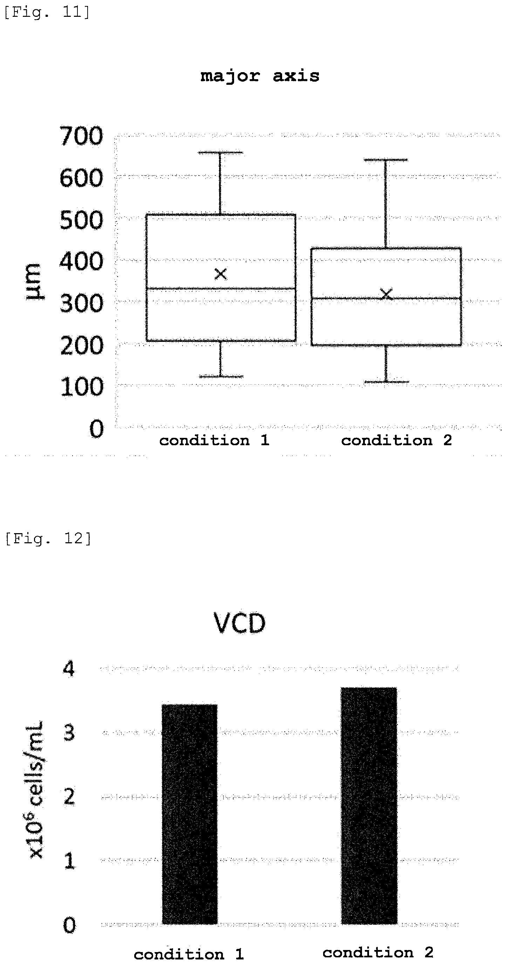

   Fig. 11 shows the influence of the concentration of Ca²⁺ in the medium on the size of iPS cell aggregates.
[Fig. 12]
   Fig. 12 shows the influence of the concentration of Ca²⁺ in the medium on the viable cell density (VCD) of iPS cells.
[Fig. 13]

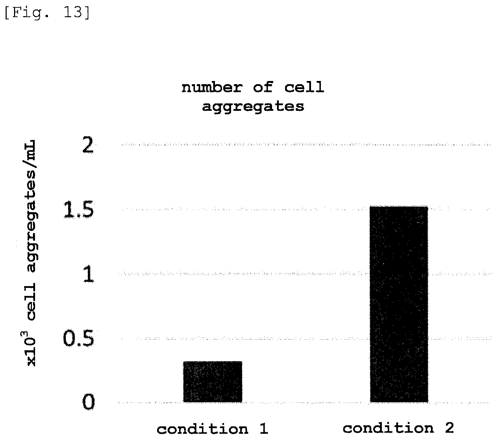

   Fig. 13 shows the influence of the concentration of Ca²⁺ in the medium on the number of iPS cell aggregates.

### [Description of Embodiments]

The present invention is explained in detail in the following.

### Definition

In the present specification, the "suspension culture" refers to a cell culture method performed in a state where cells do not adhere to the culture container. In the present invention, the suspension culture may or may not be accompanied by pressure from the outside or vibration on the liquid medium, or shaking or rotation operation in the liquid medium.

In the present specification, the "cell aggregate" means an aggregate of cells formed by cells adhered to each other when subjected to suspension culture. The cell aggregate is sometimes referred to as sphere (or spheroid).

In the present specification, the size of the cell aggregate is determined by measuring the major axis thereof. A method known per se can be used for measuring the size of the cell aggregates. The size of the cell aggregates can be easily measured using a commercially available device such as BZ-X710 (Keyence) and the like.

### cell culture method 1

The present invention provides a method for culturing cells, including the following steps (hereinafter sometimes referred to as "the method 1 of the present invention"):
a first step of preparing a population of cell aggregates having a major axis of not more than 400 µm, and
a second step of suspension culturing the population of cell aggregates obtained in the first step.

The first step in the method 1 of the present invention aims to prepare a population of cell aggregates with a comparatively small size.

In the method 1 of the present invention, the upper limit of the size of the cell aggregate (i.e., major axis of cell aggregate) prepared in the first step is 400 µm. A cell aggregate having a longer major axis than this is not preferable from the aspect of cell proliferation because the cells located in the central part of the cell aggregate cannot uptake the necessary components from the medium when subjected to further suspension culture and easily undergo apoptosis. When the cells are stem cells, the cells in the central part not only easily undergo apoptosis, but also cannot maintain an undifferentiated state, which in turn creates a growing concern that a population of stem cells not uniform in quality may be produced.

In one embodiment of the method 1 of the present invention, the upper limit of the major axis of the cell aggregates prepared in the first step may be generally 400 µm, preferably 390 µm, 380 µm, 370 µm, 360 µm, 350 µm, 340 µm, 330 µm, 320 µm, 310 µm, 300 µm, 290 µm, 280 µm, 270 µm, 260 µm, 250 µm, 240 µm, 230 µm, 220 µm, 210 µm, or 200 µm.

In one embodiment of the method 1 of the present invention, the mean major axis of the cell aggregates may be not more than 400 µm, preferably not more than 300 µm, not more than 250 µm, not more than 200 µm, not more than 190 µm, not more than 180 µm, not more than 170 µm, not more than 160 µm, not more than 150 µm, not more than 140 µm, not more than 130 µm, not more than 120 µm, not more than 110 µm, not more than 100 µm, not more than 95 µm, not more than 90 µm, not more than 85 µm, or not more than 80 µm. In one embodiment, the mean major axis of the cell aggregates may be 80 to 400 µm, 80 to 300 µm, 80 to 250 µm, 80 to 200 µm, 80 to 190 µm, 80 to 180 µm, 80 to 170 µm, 80 to 160 µm, 80 to 150 µm, 80 to 140 µm, 80 to 130 µm, 80 to 120 µm, 80 to 110 µm, 80 to 100 µm, 80 to 95 µm, 80 to 90 µm, or 80 to 85 µm.

In another embodiment of the method 1 of the present invention, the median major axis of the cell aggregates may be not more than 400 µm, preferably not more than 300 µm, not more than 250 µm, not more than 200 µm, not more than 190 µm, not more than 180 µm, not more than 170 µm, not more than 160 µm, not more than 150 µm, not more than 140 µm, not more than 130 µm, not more than 120 µm, not more than 110 µm, not more than 100 µm, not more than 95 µm, not more than 90 µm, not more than 85 µm, not more than 80 µm, not more than 75 µm, or not more than 70 µm. In one embodiment, the median major axis of the cell aggregates may be 70 to 400 µm, 70 to 300 µm, 70 to 250 µm, 70 to 200 µm, 70 to 190 µm, 70 to 180 µm, 70 to 170 µm, 70 to 160 µm, 70 to 150 µm, 70 to 140 µm, 70 to 130 µm, 70 to 120 µm, 70 to 110 µm, 70 to 100 µm, 70 to 95 µm, 70 to 90 µm, 70 to 85 µm, 70 to 80 µm, or 70 to 75 µm.

In the first step of the method 1 of the present invention, any method known per se may be used as long as the size of the above-mentioned cell aggregate can be adjusted. For example, the first step of the method 1 of the present invention can be achieved by suspension culture of cells in a spinner flask. Briefly, it is known that, when a certain number of cells are subjected to suspension culture in a spinner flask, the size of the cell aggregate formed after a certain period of time becomes larger as the number of rotations of the blade becomes smaller, and smaller as the number of rotations becomes larger. Therefore, those of ordinary skill in the art can easily prepare a cell aggregate of a desired size by appropriately determining the number of rotations of the blades of the spinner flask.

The spinner flask is not particularly limited as long as it can prepare a cell aggregate having a desired size, and a commercially available spinner flask may be used. When iPS cells are used as the cells, for example, a 30 mL single-use bioreactor for iPS cells (manufactured by ABLE, model number BWV-S03A) or the like is preferably used.

Alternatively, a population of cell aggregates having the above-mentioned preferred size may also be prepared by once preparing a population of cell aggregates having a relatively large size, and dividing the cell aggregates by passing through a mesh having an appropriate pore size or the like.

In a still another embodiment, cell aggregates having a desired size may also be prepared by suspension culture using a low-adhesive plate. Examples of the low-adhesive plate used in such a method include, but are not limited to, "Corning (registered trade mark) Elplasia (registered trade mark) plate" and the like.

The mesh is not particularly limited as long as it can be sterilized, and examples thereof include a nylon mesh, a metal (e.g., stainless steel) mesh, and the like. As the pore size of the mesh in the case of, for example, nylon mesh, the opening is about 20 to 100 µm, preferably about 30 to 70 µm, more preferably about 40 to 60 µm, but the size is not limited thereto. While the shape and the like of the mesh are not particularly limited, it is preferable that the mesh has a thickness and a shape that do not damage the cells as much as possible. For example, a metal (e.g., stainless steel) mesh is preferred because the thickness of the mesh can be reduced easily and therefore the damage on the cells during the division is relatively small.

In another embodiment, a cell aggregate having a desired size may also be prepared by adjusting the concentration of calcium ion (Ca²⁺) in the medium. In another embodiment, a cell aggregate having a desired size may also be prepared by adjusting the concentration of calcium ion (Ca²⁺) and magnesium ion (Mg²⁺) in the medium.

The concentration of calcium ion in the medium used in the step of preparing a cell aggregate of a relatively small size (that is, the first step) is not particularly limited as long as a cell aggregate of a desired size can be prepared, and may be, for example, the following concentration range:
(1) Ca²⁺=0.07 to 0.25 mM
(2) Ca²⁺=0.10 to 0.25 mM
(3) Ca²⁺=0.13 to 0.25 mM
(4) Ca²⁺=0.18 to 0.25 mM
(5) Ca²⁺=0.20 to 0.25 mM.

When the concentrations of the calcium ion and the magnesium ion are adjusted, the concentration ranges of the calcium ion and the magnesium ion are not particularly limited as long as a cell aggregate of a desired size can be prepared, and may be, for example, the following concentration range:
(6) Ca²⁺/Mg²⁺=0.07 to 0.25 mM/0.5 to 0.65 mM
(7) Ca²⁺/Mg²⁺=0.10 to 0.25 mM/0.5 to 0.65 mM
(8) Ca²⁺/Mg²⁺=0.13 to 0.25 mM/0.5 to 0.65 mM
(9) Ca²⁺/Mg²⁺=0.18 to 0.25 mM/0.5 to 0.65 mM
(10) Ca²⁺/Mg²⁺=0.20 to 0.25 mM/0.5 to 0.65 mM.

The embodiments of the first step are not limited to the aforementioned two forms, and a method of dividing cell aggregates by using an enzyme instead of the mesh, and the like can also be used.

In one embodiment of the method 1 of the present invention, the population of the cell aggregates in the first step is prepared using a spinner flask. The use of a spinner flask is preferred because it is simple and inexpensive, and the physical/chemical damage on the cell aggregates can be minimized.

The second step of the method 1 of the present invention aims to further proliferate the cells by subjecting the population of cell aggregates obtained in the first step to suspension culture.

The suspension culture in the second step is not particularly limited as long as the cells constituting the cell aggregates can proliferate, and a suspension culture method known per se may be used. In one embodiment, suspension culture can be performed under known conditions and using a commercially available bioreactor and the like.

In one embodiment, it is preferable to adjust the concentration of calcium ion in the medium used in the second step of the method 1 of the present invention. The concentration range of calcium ion in the medium used in second step may be, for example, the following concentration range:
(1) Ca²⁺=0.07 to 0.25 mM
(2) Ca²⁺=0.10 to 0.25 mM
(3) Ca²⁺=0.13 to 0.25 mM
(4) Ca²⁺=0.18 to 0.25 mM
(5) Ca²⁺=0.20 to 0.25 mM.

In another embodiment, the concentration range of calcium ion in the medium used in the second step of the method 1 of the present invention may be, for example, the following concentration range:
(6) Ca²⁺=0.07 to 0.25 mM
(7) Ca²⁺=0.07 to 0.22 mM
(8) Ca²⁺=0.07 to 0.20 mM
(9) Ca²⁺=0.07 to 0.18 mM
(10) Ca²⁺=0.07 to 0.15 mM.

Similar to the first step, the concentrations of the calcium ion and the magnesium ion in the medium used in the second step may be adjusted. Preferred concentration ranges of the calcium ion and the magnesium ion in the second step are not particularly limited as long as the desired effect of the present invention is obtained, and may be, for example, the following concentration range:
(1) Ca²⁺/Mg²⁺=0.07 to 0.25 mM/0.5 to 0.65 mM
(2) Ca²⁺/Mg²⁺=0.10 to 0.25 mM/0.5 to 0.65 mM
(3) Ca²⁺/Mg²⁺=0.13 to 0.25 mM/0.5 to 0.65 mM
(4) Ca²⁺/Mg²⁺=0.18 to 0.25 mM/0.5 to 0.65 mM
(5) Ca²⁺/Mg²⁺=0.20 to 0.25 mM/0.5 to 0.65 mM.

In one embodiment of the method 1 of the present invention, it is also preferable to set the concentrations of the calcium ion and/or the magnesium ion in the medium used in the first step to the calcium concentration and/or the magnesium ion concentration of a medium used generally (e.g., calcium ion: 0.8 to 1.2 mM, magnesium ion: 0.8 to 1.2 mM) without reducing to the above-mentioned ranges, and set only the concentrations of the calcium ion and/or the magnesium ion in the medium used in the second step to fall within the above-mentioned concentration ranges.

In another embodiment of the method 1 of the present invention, the first step and the second step may be performed successively. More specifically, when a desired high density culture can be achieved by preparing a large number of cell aggregates having a relatively small size in a certain culture system and successively conducting the culture as it is without changing the culture system, it is not necessary to set a clear second step, and the first step may be continuously performed to replace the second step.

The cell type to which the method 1 of the present invention can be applied is not particularly limited as long as it is a cell that forms a sphere when subjected to suspension culture. Examples of such cell type include germ cells such as spermatozoon, ovum, and the like, somatic cells constituting the living body, stem cells (pluripotent stem cell, etc.), precursor cells, cancer cells separated from the living body, cells (cell lines) that are separated from the living body, acquire immortalizing ability, and are stably maintained ex-vivo, cells that are separated from the living body and subjected to artificial gene modification, cells that are separated from the living body and subjected to artificial nuclear exchange, and the like. Examples of the somatic cell constituting the living body include, but are not limited to, fibroblast, bone marrow cell, B lymphocyte, T lymphocyte, neutrophil, erythrocyte, platelet, macrophage, monocyte, osteocyte, pericyte, dendritic cell, keratinocyte, adipocyte, mesenchymal cell, epithelial cell, epidermis cell, endothelial cell, vascular endothelial cell, hepatocyte, chondrocyte, cumulus cell, neuronal cells, glial cell, neuron, oligodendrocyte, micro glia, astrocyte, heart cell, esophageal cell, muscle cells (e.g., smooth myocyte or skeleton myocyte), pancreas beta cell, melanocyte, hematopoietic progenitor cell (e.g., CD34 positive cell derived from cord blood), mononuclear cell, and the like. The somatic cell includes, for example, cells taken from any tissue such as skin, kidney, spleen, adrenal gland, liver, lung, ovary, pancreas, uterus, stomach, colon, small intestine, large intestine, bladder, prostate, testis, thymus, muscle, connective tissue, bone, cartilage, vascular tissue, blood (including cord blood), bone marrow, heart, eye, brain, neural tissue, and the like.

Stem cell is a cell that has the ability to replicate itself and the ability to differentiate into other multi-lineage cells. Examples thereof include, but are not limited to, embryonic stem cell (ES cell), embryonal carcinoma cell, embryonic germ cell, induced pluripotent stem cell (iPS cell), neural stem cell, hematopoietic stem cell, mesenchymal stem cell, hepatic stem cell, pancreatic stem cell, muscle stem cell, germ stem cell, intestinal stem cell, cancer stem cell, hair follicle stem cell, and the like.

A cell line is a cell that has acquired infinite proliferation potency through artificial manipulation outside the body. Examples thereof include, but are not limited to, CHO (Chinese hamster ovary cell line), HCT116, Huh7, HEK293 (human fetal kidney cell), HeLa (human uterine cancer cell line), HepG2 (human liver cancer cell line), UT7/TPO (human leukemia cell line), MDCK, MDBK, BHK, C-33A, HT-29, AE-1, 3D9, Ns0/1, Jurkat, NIH3T3, PC12, S2, Sf9, Sf21, High Five (registered trade mark), Vero, and the like.

In one preferred embodiment of the method 1 of the present invention, the cell is a stem cell, more preferably an iPS cell.

The medium used in the first step and the second step in the method 1 of the present invention is not particularly limited as long as cell proliferation can be achieved. The media to be used may be the same or different in the first step and the second step. The medium to be used may be prepared by a method known per se according to the cells to be cultured, or a commercially available product.

Examples of the medium to be used include Dulbecco's modified Eagle medium (DMEM), Ham's Nutrient Mixture F12, DMEM/F12 medium, McCoy's 5A medium, Minimum Essential medium (MEM), Eagle's Minimum Essential medium (EMEM), alpha Modified Eagle's Minimum Essential medium (αMEM), Roswell Park Memorial Institute (RPMI) 1640 medium, Iscove's Modified Dulbecco's medium (IMDM), MCDB131 medium, William's medium E, Fischer's medium, and the like.

Examples of the medium to be particularly used for culturing stem cells include STEMPRO (registered trade mark) hESC SFM medium (Life Technologies), mTeSR1 medium (STEMCELL Technologies), TeSR2 medium (STEMCELL Technologies), TeSR-E8 medium (STEMCELL Technologies), Essential 8 medium (Life Technologies), HEScGRO (trade mark) Serum-Free medium for hES cells (Millipore), PluriSTEM (trade mark) Human ES/iPS medium (EMD Millipore), NutriStem (registered trade mark) hESC XF medium (Biological Industries Israel Beit-Haemek), NutriStem (trade mark) XF/FF Culture medium (Stemgent), AF NutriStem (registered trade mark) hESC XF medium (Biological Industries Israel Beit-Haemek), S-medium (DS pharma biomedical), StemFit (registered trade mark) AK03N medium (Ajinomoto Co., Inc.), hESF9 medium, hESF-FX medium, CDM medium, DEF-CS 500 Xeno-Free 3D Spheroid Culture medium (Cellartis), StemFlex medium (Thermo Fisher Scientific), and the like.

In one embodiment of the method 1 of the present invention, components preferable for cell proliferation can also be further added to the medium used in the first step and/or the second step. Examples of such component include sugars such as glucose, fructose, sucrose, maltose, and the like; amino acids such as asparagine, aspartic acid, glutamine, glutamic acid, and the like; proteins such as albumin, transferrin, and the like; peptides such as glycylglycylglycine, soybean peptide, and the like; serum; vitamins such as choline, vitamin A, vitamin Bs (thiamine, riboflavin, pyridoxine, cyanocobalamin, biotin, folic acid, pantothenic acid, nicotine amide etc.), vitamin C, vitamin E, and the like; fatty acids such as oleic acid, arachidonic acid, linoleic acid, and the like; lipids such as cholesterol and the like; inorganic salts such as sodium chloride, potassium chloride, calcium chloride, magnesium sulfate, sodium dihydrogen phosphate, and the like; trace elements such as zinc, copper, selenium, and the like; buffering agents such as N,N-bis(2-hydroxyethyl)-2-aminoethanesulfonic acid (BES), 4-(2-hydroxyethyl)-1-piperazineethanesulfonic acid (HEPES), N-[tris(hydroxymethyl)methyl]glycine (Tricine), and the like; antibiotics such as amphotericin B, kanamycin, gentamicin, streptomycin, penicillin, and the like; cell adhesion factors and extracellular matrix components such as Type I collagen, Type II collagen, fibronectin, laminin, poly-L-lysine, poly-D-lysine, and the like; cytokines and growth factors such as interleukin, fibroblast growth factor (FGF), hepatocyte growth factor (HGF), transforming growth factor (TGF)-α, transforming growth factor (TGF)-β, vascular endothelium growth factor (VEGF), activin A, and the like; hormones such as dexamethasone, hydrocortisone, estra diol, progesterone, glucagon, insulin, and the like; and the like. Appropriate components can be selected and used according to the type of the cells to be cultured.

In one preferred embodiment of the method 1 of the present invention, D-glucose and five kinds of amino acids (tryptophan, serine, cysteine (or cystine), methionine, arginine) may be further added to the medium used in the first step and/or the second step.

Glucose (or a salt thereof) can be added to the medium such that the converted glucose concentration is generally 0.1 g/L/day to 900 g/L/day, preferably 1 g/L/day to 200 g/L/day, more preferably 1 g/L/day to 20 g/L/day.

In addition, five kinds of amino acids (tryptophan, serine, cysteine (cystine), methionine, and arginine) can be added to the medium of the present invention such that the concentration of tryptophan (concentration after conversion to tryptophan in a free form) is generally 0.1 mg/L/day to 11000 mg/L/day, preferably 1 mg/L/day to 1000 mg/L/day, more preferably 1 mg/L/day to 100 mg/L/day, the concentration of serine (concentration after conversion to serine in a free form) is generally 0.1 mg/L/day to 425000 mg/L/day, preferably 1 mg/L/day to 1000 mg/L/day, more preferably 1 mg/L/day to 100 mg/L/day, the concentration of cysteine or cystine (concentration after conversion to cysteine in a free form) is generally 0.1 mg/L/day to 280000 mg/L/day, preferably 1 mg/L/day to 1000 mg/L/day, more preferably 1 mg/L/day to 100 mg/L/day, the concentration of methionine (concentration after conversion to methionine in a free form) is generally 0.1 mg/L/day to 55000 mg/L/day, preferably 1 mg/L/day to 1000 mg/L/day, more preferably 1 mg/L/day to 100 mg/L/day, and the concentration of arginine (concentration after conversion to arginine in a free form) is generally 0.1 mg/L/day to 150000 mg/L/day, preferably 1 mg/L/day to 2000 mg/L/day, more preferably 1 mg/L/day to 200 mg/L/day.

When the method 1 of the present invention is applied to stem cells, in one preferred embodiment, bFGF may be further added at regular intervals to the medium used in the first step and/or the second step.

In the method 1 of the present invention, as the cell culture condition, a method known per se may be selected according to the cell type. For example, the culture temperature may be generally 25°C to 39°C, preferably 33°C to 39°C. The carbon dioxide concentration may be generally 4% by volume to 10% by volume, preferably 4% by volume to 6% by volume. The oxygen concentration may be generally 1% by volume to 25% by volume, preferably 4% by volume to 20% by volume. The frequency of medium exchange may be once every two to three days, once a day, or multiple times a day (e.g., twice). In addition, the whole medium may be exchanged or a part (e.g., 10%, 20%, 30%, 40%, 50%, 60%, 70%, 80%, or 90%, etc.) thereof may be exchanged at the time of exchange. The amount of the medium to be exchanged during the medium exchange may be appropriately determined according to the cell type and the like.

### cell culture method 2

The present invention also provides a method for culturing cells, including a step of suspension culturing cells in a medium having a calcium ion concentration of 0.07 to 0.25 mM (hereinafter sometimes referred to as "the method 2 of the present invention").

The cell type to which the method 2 of the present invention can be applied is not particularly limited as long as it is a cell that forms a sphere when subjected to suspension culture. Examples of such cell type include germ cells such as spermatozoon, ovum, and the like, somatic cells constituting the living body, stem cells (pluripotent stem cell, etc.), precursor cells, cancer cells separated from the living body, cells (cell lines) that are separated from the living body, acquire immortalizing ability, and are stably maintained ex-vivo, cells that are separated from the living body and subjected to artificial gene modification, cells that are separated from the living body and subjected to artificial nuclear exchange, and the like. Examples of the somatic cell constituting the living body include, but are not limited to, fibroblast, bone marrow cell, B lymphocyte, T lymphocyte, neutrophil, erythrocyte, platelet, macrophage, monocyte, osteocyte, pericyte, dendritic cell, keratinocyte, adipocyte, mesenchymal cell, epithelial cell, epidermis cell, endothelial cell, vascular endothelial cell, hepatocyte, chondrocyte, cumulus cell, neuronal cells, glial cell, neuron, oligodendrocyte, micro glia, astrocyte, heart cell, esophageal cell, muscle cells (e.g., smooth myocyte or skeleton myocyte), pancreas beta cell, melanocyte, hematopoietic progenitor cell (e.g., CD34 positive cell derived from cord blood), mononuclear cell, and the like. The somatic cell includes, for example, cells taken from any tissue such as skin, kidney, spleen, adrenal gland liver, lung, ovary, pancreas, uterus, stomach, colon, small intestine, large intestine, bladder, prostate, testis, thymus, muscle, connective tissue, bone, cartilage, vascular tissue, blood (including cord blood), bone marrow, heart, eye, brain, neural tissue, and the like.

Stem cell is a cell that has the ability to replicate itself and the ability to differentiate into other multi-lineage cells. Examples thereof include, but are not limited to, embryonic stem cell (ES cell), embryonal carcinoma cell, embryonic germ cell, induced pluripotent stem cell (iPS cell), neural stem cell, hematopoietic stem cell, mesenchymal stem cell, hepatic stem cell, pancreatic stem cell, muscle stem cell, germ stem cell, intestinal stem cell, cancer stem cell, hair follicle stem cell, and the like.

A cell line is a cell that has acquired infinite proliferation potency through artificial manipulation outside the body. A cell line is a cell that has acquired infinite proliferation potency through artificial manipulation outside the body. Examples thereof include, but are not limited to, CHO (Chinese hamster ovary cell line), HCT116, Huh7, HEK293 (human fetal kidney cell), HeLa (human uterine cancer cell line), HepG2 (human liver cancer cell line), UT7/TPO (human leukemia cell line), MDCK, MDBK, BHK, C-33A, HT-29, AE-1, 3D9, Ns0/1, Jurkat, NIH3T3, PC12, S2, Sf9, Sf21, High Five (registered trade mark), Vero, and the like.

In one preferred embodiment of the method 2 of the present invention, the cell is a stem cell, more preferably an iPS cell.

Examples of the medium used in the method 2 of the present invention include Dulbecco's modified Eagle medium (DMEM), Ham's Nutrient Mixture F12, DMEM/F12 medium, McCoy's 5A medium, Minimum Essential medium (MEM), Eagle's Minimum Essential medium (EMEM), alpha Modified Eagle's Minimum Essential medium (αMEM), Roswell Park Memorial Institute (RPMI) 1640 medium, Iscove's Modified Dulbecco's medium (IMDM), MCDB131 medium, William's medium E, Fischer's medium, and the like.

Examples of the medium particularly used for culturing stem cells include STEMPRO (registered trade mark) hESC SFM medium (Life Technologies), mTeSR1 medium (STEMCELL Technologies), TeSR2 medium (STEMCELL Technologies), TeSR-E8 medium (STEMCELL Technologies), Essential 8 medium (Life Technologies), HEScGRO (trade mark) Serum-Free medium for hES cells (Millipore), PluriSTEM (trade mark) Human ES/iPS medium (EMD Millipore), NutriStem (registered trade mark) hESC XF medium (Biological Industries Israel Beit-Haemek), NutriStem (trade mark) XF/FF Culture medium (Stemgent), AF NutriStem (registered trade mark) hESC XF medium (Biological Industries Israel Beit-Haemek), S-medium (DS pharma biomedical), StemFit (registered trade mark) AK03N medium (Ajinomoto Co., Inc.), hESF9 medium, hESF-FX medium, CDM medium, DEF-CS 500 Xeno-Free 3D Spheroid Culture medium (Cellartis), StemFlex medium (Thermo Fisher Scientific), and the like.

In one embodiment of the method 2 of the present invention, components preferable for cell proliferation can also be further added to the medium to be used. Examples of such component include sugars such as glucose, fructose, sucrose, maltose, and the like; amino acids such as asparagine, aspartic acid, glutamine, glutamic acid, and the like; proteins such as albumin, transferrin, and the like; peptides such as glycylglycylglycine, soybean peptide, and the like; serum; vitamins such as choline, vitamin A, vitamin Bs (thiamine, riboflavin, pyridoxine, cyanocobalamin, biotin, folic acid, pantothenic acid, nicotine amide etc.), vitamin C, vitamin E, and the like; fatty acids such as oleic acid, arachidonic acid, linoleic acid, and the like; lipids such as cholesterol and the like; inorganic salts such as sodium chloride, potassium chloride, calcium chloride, magnesium sulfate, sodium dihydrogen phosphate, and the like; trace elements such as zinc, copper, selenium, and the like; buffering agents such as N,N-bis(2-hydroxyethyl)-2-aminoethanesulfonic acid (BES), 4-(2-hydroxyethyl)-1-piperazineethanesulfonic acid (HEPES), N-[tris(hydroxymethyl)methyl]glycine (Tricine), and the like; antibiotics such as amphotericin B, kanamycin, gentamicin, streptomycin, penicillin, and the like; cell adhesion factors and extracellular matrix components such as Type I collagen, Type II collagen, fibronectin, laminin, poly-L-lysine, poly-D-lysine, and the like; cytokines and growth factors such as interleukin, fibroblast growth factor (FGF), hepatocyte growth factor (HGF), transforming growth factor (TGF)-α, transforming growth factor (TGF)-β, vascular endothelium growth factor (VEGF), activin A, and the like; hormones such as dexamethasone, hydrocortisone, estra diol, progesterone, glucagon, insulin, and the like; and the like. Appropriate components can be selected and used according to the type of the cells to be cultured.

The method 2 of the present invention is characterized by appropriate adjustment of the concentration of calcium ion contained in the medium. The range of the concentration of calcium ion contained in the medium to be used is, though not limited to, generally 0.07 to 0.25 mM, preferably 0.10 to 0.25 mM, more preferably 0.13 to 0.25 mM, further preferably 0.18 to 0.25 mM, particularly preferably 0.20 to 0.25 mM.

In another embodiment, in the method 2 of the present invention, the range of the concentration of calcium ion contained in the medium is, though not limited to, generally 0.07 to 0.25 mM, preferably 0.07 to 0.22 mM, more preferably 0.07 to 0.20 mM, further preferably 0.07 to 0.18 mM, particularly preferably 0.07 to 0.15 mM.

In one embodiment, the calcium ion concentration and the magnesium ion concentration may be adjusted. The range of the concentrations of calcium ion and magnesium ion contained in the medium is not particularly limited as long as the desired effect is obtained, and may be the following:
(1) Ca²⁺/Mg²⁺=0.07 to 0.25 mM/0.5 to 0.65 mM
(2) Ca²⁺/Mg²⁺=0.10 to 0.25 mM/0.5 to 0.65 mM
(3) Ca²⁺/Mg²⁺=0.13 to 0.25 mM/0.5 to 0.65 mM
(4) Ca²⁺/Mg²⁺=0.18 to 0.25 mM/0.5 to 0.65 mM
(5) Ca²⁺/Mg²⁺=0.20 to 0.25 mM/0.5 to 0.65 mM.

In another embodiment, the range of the concentrations of calcium ion and magnesium ion contained in the medium is not particularly limited as long as the desired effect is obtained, and may be the following:
(6) Ca²⁺/Mg²⁺=0.07 to 0.25 mM/0.5 to 0.65 mM
(7) Ca²⁺/Mg²⁺=0.07 to 0.22 mM/0.5 to 0.65 mM
(8) Ca²⁺/Mg²⁺=0.07 to 0.20 mM/0.5 to 0.65 mM
(9) Ca²⁺/Mg²⁺=0.07 to 0.18 mM/0.5 to 0.65 mM
(10) Ca²⁺/Mg²⁺=0.07 to 0.15 mM/0.5 to 0.65 mM.

In one preferred embodiment of the method 2 of the present invention, D-glucose and five kinds of amino acids (tryptophan, serine, cysteine (or cystine), methionine, arginine) may be further added to the medium to be used.

Glucose (or a salt thereof) can be added to the medium such that the converted glucose concentration is generally 0.1 g/L/day to 900 g/L/day, preferably 1 g/L/day to 200 g/L/day, more preferably 1 g/L/day to 20 g/L/day.

In addition, five kinds of 5 amino acids (tryptophan, serine, cysteine (cystine), methionine, and arginine) can be added to the medium such that the concentration of tryptophan (concentration after conversion to tryptophan in a free form) is generally 0.1 mg/L/day to 11000 mg/L/day, preferably 1 mg/L/day to 1000 mg/L/day, more preferably 1 mg/L/day to 100 mg/L/day, the concentration of serine (concentration after conversion to serine in a free form) is generally 0.1 mg/L/day to 425000 mg/L/day, preferably 1 mg/L/day to 1000 mg/L/day, more preferably 1 mg/L/day to 100 mg/L/day, the concentration of cysteine or cystine (concentration after conversion to cysteine in a free form) is generally 0.1 mg/L/day to 280000 mg/L/day, preferably 1 mg/L/day to 1000 mg/L/day, more preferably 1 mg/L/day to 100 mg/L/day, the concentration of methionine (concentration after conversion to methionine in a free form) is generally 0.1 mg/L/day to 55000 mg/L/day, preferably 1 mg/L/day to 1000 mg/L/day, more preferably 1 mg/L/day to 100 mg/L/day, and the concentration of arginine (concentration after conversion to arginine in a free form) is generally 0.1 mg/L/day to 150000 mg/L/day, preferably 1 mg/L/day to 2000 mg/L/day, more preferably 1 mg/L/day to 200 mg/L/day.

When the method 2 of the present invention is applied to stem cells, in one preferred embodiment, bFGF may be further added at regular intervals to the medium to be used.

In the method 2 of the present invention, as the cell culture condition, a method known per se may be selected according to the cell type. For example, the culture temperature may be generally 25°C to 39°C, preferably 33°C to 39°C. The carbon dioxide concentration may be generally 4% by volume to 10% by volume, preferably 4% by volume to 6% by volume. The oxygen concentration may be generally 1% by volume to 25% by volume, preferably 4% by volume to 20% by volume. The frequency of medium exchange may be once every two to three days, once a day, or multiple times (e.g., twice) a day. In addition, the whole medium may be exchanged or a part (e.g., 10%, 20%, 30%, 40%, 50%, 60%, 70%, 80%, or 90%, etc.) thereof may be exchanged at the time of exchange. The amount of the medium to be exchanged during the medium exchange may be appropriately determined according to the cell type and the like.

In the method 2 of the present invention, the cell seeding concentration at the time of start of the culture is, though not limited to, generally 1×10⁴ cells/mL to 1×10⁷ cells/mL, preferably 5×10⁴ cells/mL to 5×10⁶ cells/mL, more preferably 5×10⁴ cells/mL to 1×10⁶ cells/mL, particularly preferably 1×10⁵ cells/mL to 1×10⁶ cells/mL.

The present invention is explained in more detail in the following Examples; however, the present invention is not limited by these Examples. The particle size standard in the present specification and the Examples is the "number - weighted".

### [Example]

### [Example 1] Sphere formation and cell proliferation of iPS cells using spinner flask

Using a 30 mL single use bioreactor for iPS cells (ABLE: model number BWV-S03A), iPS cell 1231A3 strain was seeded in StemFit (registered trade mark) AK03N+10 uM Y-27632 (Wako: 034-24024) at a cell density of 6×10⁵ cells/mL and the cells were cultured with stirring in a CO₂ incubator under the conditions of 37°C, CO₂ concentration=5%, stirring speed=120 rpm. On the second day of seeding, 70% of the medium was replaced with StemFit AK03N. On the third day of seeding, the cell suspension (10 mL) was resuspended in fresh StemFit AK03N+200 ng/mL bFGF (Peprotech). The cells were transferred to a bioreactor ambr15 (sartorius: model number 001-0881), and stirring culture was continued under the conditions of 37°C, pH=7.2, dissolved oxygen concentration=4%, stirring speed=300 rpm. In addition, the major axis of the cell aggregates was quantified using BZ-X710 (Keyence) (cutoff value was set to 50 µm for the purpose of measuring the major axis of cell aggregates alone as the target). 70% of the medium was exchanged twice a day with StemFit AK03N+200 ng/mL bFGF, and 40 mg/L/day Trp (Ajinomoto Co., Inc.), 40 mg/L/day Ser (Ajinomoto Co., Inc.), 40 mg/L/day Cys hydrochloride (Japan protein), 40 mg/L/day Met (Ajinomoto Co., Inc.), 160 mg/L/day Arg (Ajinomoto Co., Inc.), and 4 g/L/day D-glucose (Nacalai Tesque, model number 16806-25) were further added to the medium. After the 5th day of culture, the number of viable cells was measured using Vi-CELL^{™} XR (Beckman Coulter), a live/dead cell autoanalyzer.

The verification results of the proliferation results of iPS cells in n=1 are shown in Fig. 1. The verification results in quadruplicate of the distribution of the major axis of the cell aggregates when transferred to a bioreactor on day 3 are shown in Fig. 2 and the following Table 1. It was shown that high density culture exceeding 1.0×10⁷ cells/mL can be realized by controlling the major axis of the cell aggregates to a size of not more than 400 µm.

**[Table 1]**

| | **Flask1** | **Flask2** | **Flask3** | **Flask4** |
|---|---|---|---|---|
| **maximum value (µm)** | **267** | **388** | **323** | **257** |
| **mean (µm)** | **92** | **98** | **100** | **93** |
| **median value (µm)** | **83** | **89** | **89** | **87** |
| **standard deviation (µm)** | **36** | **45** | **44** | **31** |

### [Example 2] Influence of presence or absence of control of sphere size on proliferation

To confirm that the control of the sphere size is important for cell proliferation, culture was performed in a micro bioreactor without controlling the sphere size. That is, iPS cell 1210B2 strain was seeded in a micro bioreactor ambr15 at a cell density of 6×10⁵ cells/mL, and the cells were cultured with stirring in a CO₂ incubator using StemFit AK03N+10uM Y-27632 under the conditions of 37°C, CO₂ concentration=5%, stirring speed=300 rpm. On the second day of seeding, 70% of the medium was replaced with StemFit AK03N. On the third day of seeding, the cell suspension (10 mL) was resuspended in fresh StemFit AK03N. Thereafter, 70% of the medium was exchanged twice a day with StemFit AK03N, and 40 mg/L/day Trp, 40 mg/L/day Ser, 40 mg/L/day Cys, 40 mg/L/day Met, 160 mg/L/day Arg, and 4 g/L/day D-glucose were further added. On the other hand, an operation similar to that in Example 1 was performed using StemFit AK03N in the group with a controlled sphere size. On the third day of culture, the major axis of the spheres was quantified using BZ-X710. After the 7th day of culture, the number of viable cells was measured using Vi-CELL (trade mark) XR, a live/dead cell autoanalyzer.

The verification results of the proliferation results of iPS cells in n=1 are shown in Fig. 3. The verification results in n=1 of the distribution of the major axis of the spheres when transferred to a micro bioreactor on day 3 are shown in Fig. 4. It was shown that 1.0×10⁷ cells/mL cannot be reached even under similar culture conditions unless the sphere size is controlled.

### [Example 3] Study of method for controlling sphere size

In the following Experimental Examples, the components that can keep the sphere size small when proliferating induced pluripotent stem cells (iPS cells) by suspension culture was studied. As the iPS cell, 1231A3 strain purchased from iPS Academia Japan was used. In addition, as a medium for iPS cells, a commercially available StemFit AK03N (Ajinomoto Co., Inc.) was used.

Using a 30 mL single use bioreactor for iPS cells (ABLE: BWV-S03A), the concentrations of calcium chloride, magnesium sulfate, and magnesium chloride were adjusted, and iPS cell 1231A3 strain was seeded in StemFit AK03N+10 uM Y-27632 (Wako: 034-24024), adjusted to the three conditions shown in Table 2, at a cell density of 2×10⁵ cells/mL and the cells were cultured with stirring in a CO₂ incubator under the conditions of 37°C, CO₂ concentration=5%, stirring speed=55 rpm. After the second day of seeding, 70% of the medium was replaced every day with StemFit AK03N under respective conditions. On the 7th day of culture, the number of viable cells was measured using Vi-CELL^{™} XR (Beckman Coulter), a live/dead cell autoanalyzer. In addition, the major axis of the cell aggregates was quantified using BZ-X710 (Keyence).

**[Table 2]**

| | **Ca2+** | **Mg2+** | |
|---|---|---|---|
| **condition 1** | 0.844 | 1.003 | |
| **condition 2** | 0.214 | 0.579 | |
| **condition 3** | 0.144 | 0.532 | (mM) |

The verification results of the proliferation results of iPS cells in n=1 are shown in Fig. 5. In addition, the verification results in n=1 of the distribution of the major axis of the spheres are shown in Fig. 6 and Table 3. It was shown that the major axis of the spheres can be maintained small by adjusting the concentrations of Ca²⁺ and Mg^{2+.}

**[Table 3]**

| | **condition 1** | **condition 2** | **condition 3** |
|---|---|---|---|
| **maximum value** | 425 | 374 | 323 |
| **third quartile** | 271.75 | 210 | 222.25 |
| **median value** | 212 | 153 | 150 |
| **first quartile** | 151.75 | 107 | 112 |
| **minimum value** | 52 | 54 | 55 |
| **average** | 208 | 161 | 162 |

### [Example 4] Study of influence of magnesium ion and calcium ion concentration on cell proliferation

Using a 30 mL single use bioreactor for iPS cells (ABLE: BWV-S03A), iPS cell 1210B2 strain was seeded in StemFit AK03N+10 µM Y-27632 (Wako: 034-24024) at a cell density of 6×10⁵ cells/mL and the cells were cultured with stirring in a CO₂ incubator under the conditions of 37°C, CO₂ concentration=5%, stirring speed=120 rpm. On the second day of seeding, 70% of the medium was replaced with StemFit AK03N. On the third day of seeding, the cell suspension (10 mL) was resuspended in fresh StemFit AK03N with adjusted concentrations of calcium chloride, magnesium sulfate, and magnesium chloride, and adjusted to the two conditions shown in Table 4. The cells were transferred to a micro bioreactor ambr15 (sartorius: 001-0881), and stirring culture was continued under the conditions of 37°C, pH=7.2, dissolved oxygen concentration=20%, stirring speed=300 rpm. 70% of the medium was exchanged twice a day with StemFit AK03N under respective conditions, and 40 mg/L/day Trp (Ajinomoto Co., Inc.), 40 mg/L/day Ser (Ajinomoto Co., Inc.), 40 mg/L/day Cys hydrochloride (Japan protein), 40 mg/L/day Met (Ajinomoto Co., Inc.), 160 mg/L/day Arg (Ajinomoto Co., Inc.), 4 g/L/day D-glucose (Nacalai Tesque: 16806-25) were further added to both groups. On the 7th day of culture, the number of viable cells was measured using Vi-CELL^{™} XR (Beckman Coulter), a live/dead cell autoanalyzer.

**[Table 4]**

| | **Ca2+** | **Mg2+** | |
|---|---|---|---|
| **condition 1** | 0.844 | 1.003 | |
| **condition 2** | 0.064 | 0.497 | (mM) |

The verification results in duplicate of the influence of Ca²⁺ and Mg²⁺ on the proliferation of iPS cells are shown in Fig. 7. As shown in Fig. 7, it was shown that Ca²⁺ and Mg²⁺ have a great influence on cell proliferation at certain concentrations.

### [Example 5] Study of method for controlling sphere size - 2

In the aforementioned Example 3, it was shown that the concentrations of Ca²⁺ and Mg²⁺ are important for controlling the major axis of spheres. In this Example, which of the concentrations of Ca²⁺ and Mg²⁺ influences more on the control of the major axis of spheres was studied.

Using a 30 mL single use bioreactor for iPS cells (ABLE: BWV-S03A), iPS cell 1210B2 strain was seeded in StemFit AK03N+10 uM Y-27632 (Wako: 034-24024) at a cell density of 6×10⁵ cells/mL and the cells were cultured with stirring in a CO₂ incubator under the conditions of 37°C, CO₂ concentration=5%, stirring speed=120 rpm. On the second day of seeding, 70% of the medium was replaced with StemFit AK03N. (The calcium ion concentration of the medium used up to this point was not adjusted. Therefore, the calcium ion concentration of the medium was of the same level as the calcium ion concentration of a general cell culture medium (about 0.8 mM to 1.2 mM).) On the third day of seeding, the cell suspension (10 mL) was resuspended in fresh StemFit AK03N with an adjusted concentration of calcium chloride, and adjusted to the two conditions shown in Table 5. The cells were transferred to a micro bioreactor ambr15 (sartorius: 001-0881), and stirring culture was continued under the conditions of 37°C, pH=7.2, dissolved oxygen concentration=20%, stirring speed=300 rpm. In conditions 1 and conditions 2, the concentration of Mg²⁺ was the same (1.003 mM). 70% of the medium was exchanged twice a day with StemFit AK03N under conditions, and 40 mg/L/day Trp (Ajinomoto Co., Inc.), 40 mg/L/day Ser (Ajinomoto Co., Inc.), 40 mg/L/day Cys hydrochloride (Japan protein), 40 mg/L/day Met (Ajinomoto Co., Inc.), 160 mg/L/day Arg (Ajinomoto Co., Inc.), 18.6 mg/L/day His (Ajinomoto Co., Inc.), 43.7 mg/L/day Ile (Ajinomoto Co., Inc.), 47.3 mg/L/day Leu (Ajinomoto Co., Inc.), 73.1 mg/L/day Lys hydrochloride (Ajinomoto Co., Inc.), 28.4 mg/L/day Phe (Ajinomoto Co., Inc.), 42.3 mg/L/day Val (Ajinomoto Co., Inc.), 4 g/L/day D-glucose (Nacalai Tesque:16806-25)) were further added to both groups. On the 3rd and 6th days of culture, the major axis of the spheres was quantified using BZ-X710 (Keyence). On the 3rd and 6th days of culture, moreover, the number of viable cells was measured using Vi-CELL (trade mark) XR (Beckman Coulter), a live/dead cell autoanalyzer.

**[Table 5]**

| | **condition 1** | **condition 2** | |
|---|---|---|---|
| **Ca**²⁺ | 0.844 | 0.144 | mM |

The verification results of the influence of Ca²⁺ concentration on the sphere size of iPS cells in n=1 are shown in Fig. 8 and Table 6. In addition, VCD is shown in Fig. 9, and the number of cell aggregates is shown in Fig. 10.

As is clear from these Figures and Table, it was shown that the major axis of spheres can be maintained small and the number of spheres can be maintained high by adjusting the Ca²⁺ concentration.

**[Table 6]**

| | **day6** | | |
|---|---|---|---|
| | **condition 1** | **condition 2** | |
| **maximum value** | 420 | 328 | µm |
| **median value** | 157 | 86 | µm |
| **minimum value** | 37 | 33 | µm |
| **mean** | 173 | 99 | µm |

### [Example 6] Influence on cell proliferation of iPS cell using suspension culture system

iPS cell 1210B2 strain was seeded at a cell density of 1.72×10⁵ cells/mL in a micro bioreactor ambr15 (sartorius: 001-0881), and the cells were cultured with stirring using fresh StemFit AK03N+10uM Y-27632 with adjusted calcium chloride concentration, and adjusted to the two conditions shown in Table 7 under the conditions of 37°C, pH=7.2, dissolved oxygen concentration=20%, stirring speed=300 rpm. After the second day of culture, 70% of the medium was exchanged twice a day with StemFit AK03N under respective conditions, and 40 mg/L/day Trp (Ajinomoto Co., Inc.), 40 mg/L/day Ser (Ajinomoto Co., Inc.), 40 mg/L/day Cys hydrochloride (Japan protein), 40 mg/L/day Met (Ajinomoto Co., Inc.), 160 mg/L/day Arg (Ajinomoto Co., Inc.), 18.6 mg/L/day His (Ajinomoto Co., Inc.), 43.7 mg/L/day Ile (Ajinomoto Co., Inc.), 47.3 mg/L/day Leu (Ajinomoto Co., Inc.), 73.1 mg/L/day Lys hydrochloride (Ajinomoto Co., Inc.), 28.4 mg/L/day Phe (Ajinomoto Co., Inc.), 42.3 mg/L/day Val (Ajinomoto Co., Inc.), and 4 g/L/day D-glucose (Nacalai Tesque:16806-25)) were further added to both groups. On the 6th day, the major axis of the spheres was quantified using BZ-X710 (Keyence). On the 6th days of culture, moreover, the number of viable cells was measured using Vi-CELL (trade mark) XR (Beckman Coulter), a live/dead cell autoanalyzer.

**[Table 7]**

| | **condition 1** | **condition 2** | |
|---|---|---|---|
| **Ca**²⁺ | 0.844 | 0.074 | mM |

The verification results of the influence of Ca²⁺ concentration on the sphere size of iPS cells in n=1 are shown in Fig. 11 and Table 8. In addition, VCD is shown in Fig. 12, and the number of cell aggregates is shown in Fig. 13. From the above, it was shown that the major axis of spheres can be maintained small and the number of spheres can be further maintained high by adjusting the Ca²⁺ concentration.

**[Table 8]**

| | **condition 1** | **condition 2** | |
|---|---|---|---|
| **maximum value** | 657 | 641 | µm |
| **median value** | 331 | 308 | µm |
| **minimum value** | 120 | 107 | µm |
| **mean** | 367 | 318 | µm |

### [Industrial Applicability]

According to the present invention, more preferable high density culture as compared with the embodiments performed previously can be realized by a highly inexpensive and simple means.

This application is based on patent application Nos. 2020-003961 filed in Japan (filing date: January 14, 2020) and 2020-096485 filed in Japan (filing date: June 2, 2020), the contents of which are incorporated in full herein.

## Claims

1. A method for culturing a cell, comprising the following steps:
a first step of preparing a population of cell aggregates having a major axis of not more than 400 µm, and
a second step of suspension culturing the population of cell aggregates obtained in the first step.

2. The method according to claim 1, wherein a mean major axis of the cell aggregate in the first step is not more than 110 µm.

3. The method according to claim 1 or 2, wherein a median major axis of the cell aggregate in the first step is not more than 90 µm.

4. The method according to any one of claims 1 to 3, wherein the first step and the second step are successively performed.

5. The method according to any one of claims 1 to 4, wherein the population of cell aggregates in the first step is prepared using a spinner flask.

6. The method according to any one of claims 1 to 5, wherein a medium used in the second step comprises a calcium ion at a concentration of 0.07 to 0.25 mM.

7. The method according to any one of claims 1 to 3, wherein the population of cell aggregates in the first step is prepared by adjusting a calcium ion concentration and the aforementioned calcium ion concentration is 0.07 to 0.25 mM.

8. The method according to claim 6 or 7, further comprising setting a magnesium ion concentration to 0.5 to 0.65 mM.

9. The method according to any one of claims 1 to 8, wherein the suspension culture in the second step is performed using a bioreactor.

10. The method according to any one of claims 1 to 9, wherein the cell is a stem cell.

11. The method according to claim 10, wherein the stem cell is an iPS cell.

12. A method for culturing cells, comprising a step of suspension culturing a cell in a medium having a calcium ion concentration of 0.07 to 0.25 mM.

13. The method according to claim 12, wherein the cell is a stem cell.

14. The method according to claim 13, wherein the stem cell is an iPS cell.
